# EUROPÄISCHE PATENTSCHRIFT

(11) **EP 2 373 615 B1**
(45) Veröffentlichungstag und Bekanntmachung des Hinweises auf die Patenterteilung: **06.11.2013**
(21) Anmeldenummer: 09760763.4
(22) Anmeldetag: 26.11.2009
(51) Int. Cl.: C07C 263/00, C07C 263/10, C07C 263/16, C07C 267/00

(54) **VERFAHREN ZUR MODIFIZIERUNG VON DIISOCYANATEN**
Method of modifying diisocyanates
Procédé destiné à la modification de diisocyanates

(30) Priorität: 03.12.2008 EP 08170623
(43) Veröffentlichungstag der Anmeldung: 12.10.2011
(73) Patentinhaber: Bayer Intellectual Property GmbH, 40789 Monheim (DE)
(72) Erfinder: RICHTER, Frank, 51373 Leverkusen (DE); HALPAAP, Reinhard, 51519 Odenthal (DE)
(74) Vertreter: BIP Patents
(86) Internationale Anmeldenummer: PCT/EP2009/008410
(87) Internationale Veröffentlichungsnummer: WO 2010/063400

(56) Entgegenhaltungen:
- EP-A- 1 422 223
- EP-A- 1 533 301
- DE-A1- 10 245 704
- LAAS H J ET AL: "ZUR SYNTHESE ALIPHATISCHER POLYISOCYANATE-LACKPOLYISOCYANATE MIT BIURET-, ISOCYANURAT- ODER URETDIONSTRUKTUR. ÖTHE SYNTHESIS OF ALIPHATIC POLYISOCYANATES CONTAINING BIURET, ISOCYANURATE OR URETDIONE BACKBONES FOR USE IN COATINGS" JOURNAL FUER PRAKTISCHE CHEMIE, WILEY VCH, WEINHEIM, Bd. 336, Nr. 3, 1. Januar 1994 (1994-01-01), Seiten 185-200, XP000441642 ISSN: 1436-9966 in der Anmeldung erwähnt

## Beschreibung

Die vorliegende Erfindung betrifft ein Verfahren zur Modifizierung von Diisocyanaten zur Herstellung von Polyisocyanaten unter Verwendung eines Katalysators, wobei die eingesetzten Diisocyanate durch ein spezielles Herstellungsverfahren erhalten werden. Die Erfindung betrifft weiterhin modifizierte Polyisocyanate sowie deren Verwendung, die aus vorstehend genannten Diisocyanaten hergestellt werden.

Bei der Herstellung von 2-Komponenten-Polyurethansystemen für Lacke und andere Beschichtungen geht man üblicherweise von Polyisocyanaten auf der Basis von Diisocyanaten zusammen mit geeigneten Polyolen aus. Für hochwertige Lacke werden bevorzugt aliphatische Diisocyanate verwendet. Aufgrund des Dampfdrucks der Diisocyanate ist es wichtig, dass der Monomergehalt in der Formulierung des Polyurethansystems möglichst niedrig ist. Daher werden die aliphatischen Diisocyanate in der Regel modifiziert, wobei Dimerisierungen und Trimerisierungen wichtige Reaktionstypen hierfür darstellen. Eine Übersicht über solche Reaktionen findet sich beispielsweise im Journal für Praktische Chemie/Chemiker-Zeitung 1994, Band 336, Seiten 185 bis 200.

Während der Herstellung von Diisocyanaten durch Phosgenierung von Diaminen fallen als Nebenprodukte chlorhaltige Verbindungen an, welche unter dem Summenparameter des hydrolysierbaren Chlors (HC, HC-Gehalt) zusammengefasst werden. Dieser Begriff wird für Verbindungen benutzt, die mit Wasser unter Bildung von Chlorwasserstoff oder Chloridionen reagieren, zum Beispiel Carbamidsäurechloride.

Die Modifizierungsreaktionen von Diisocyanaten zur Herstellung von Polyisocyanaten vom Dimer- sowie Trimertyp werden häufig in Gegenwart eines homogenen Katalysators, beispielsweise eines Phosphans, durchgeführt. So offenbart DE 103 54 544 A1 die Verwendung von speziellen Cycloalkylphosphanen als Katalysatoren für die Isocyanatdimerisierung (Uretdionbildung) und ein Verfahren zur Herstellung hoch uretdiongruppenhaltiger Polyisocyanate.

In der Vergangenheit hat sich gezeigt, dass für solche Modifizierungsreaktionen eingesetzte Diisocyanate mit einem hohen Gehalt an hydrolysierbarem Chlor zu einer Desaktivierung des Katalysators führen können, weshalb es nicht an Versuchen gefehlt hat, möglichst HC-arme beziehungsweise HC-freie Diisocyanate herzustellen und in der Modifizierung einzusetzen (siehe hierzu EP 1046637, EP 1053998).

Als Nachteil dieser Verfahrensweise sind einerseits die erhöhten Kosten zu nennen, die mit dem zusätzlichen Verfahrensschritt der HC-Absenkung einher gehen, andererseits hat es sich beim Auswirkungen unter anderem auf die Lager- und Farbstabilität der Diisocyanate haben, weshalb ihnen häufig nachträglich Stabilisatoren zugesetzt werden müssen (EP 0643042).

Aus EP 1 422 223 A1 ist ein Verfahren zur Modifizierung von Diisocyanaten zur Herstellung von Polyisocyanaten, wobei das Diisocyanate unter Verwendung eines Katalysators umgesetzt wird und wobei die Modifizierung der Diisocyanate die Bildung von Polyisocyanaten mit Uretdionstrukturen betrifft. Auch bei diesem Verfahren kann der Gehalt an hydrolisierbaren Chlorverbindungen höher sein als gewünscht.

Wünschenswert wären alternative Verfahren zur Modifizierung von Diisocyanaten, bei denen eine langsamere Desaktivierung des Katalysators eintritt.

Erfindungsgemäß vorgeschlagen wird daher ein Verfahren zur Modifizierung von Diisocyanaten zur Herstellung von Polyisocyanaten, wobei das Diisocyanat unter Verwendung eines Katalysators umgesetzt wird und wobei die Modifizierung der Diisocyanaten die Bildung von Polyisocyanaten mit Uretdion-, Isocyanurat-, Iminooxadiazindion-, Polyamid-, Carbodiimid-, Uretonimin-, Urethan-, Allophanat-, Harnstoff-, Biuret-, Amid-, Acrylharnstoff-, Imid-, Oxazolidon-, Oximcarbamat-und/oder Oxadiazintrionstrukturen betrifft.

Das Verfahren ist dadurch gekennzeichnet, dass das eingesetzte Diisocyanat erhalten wird, indem bei der Phosgenierung von Diaminen in der Gasphase das gasförmige Reaktionsgemisch gequencht wird, wobei das gasförmige Reaktionsgemisch wenigstens ein Diisocyanat, Phosgen und Chlorwasserstoff umfasst, wobei eine Quench-Flüssigkeit in das kontinuierlich aus einer Reaktionszone in eine nachgeschaltete Quench-Zone strömende Gasgemisch eingedüst wird und wobei die Quench-Flüssigkeit mit Hilfe von mindestens zwei Sprühdüsen, welche am Eingang der Quench-Zone in gleichen Abständen entlang des Umfangs der Quench-Zone angeordnet sind, eingedüst wird.

Eine erfindungsgemäße Modifizierung von Diisocyanaten beinhaltet eine Molekulargewichtserhöhung dieser Isocyanate, wobei die Molekulargewichtserhöhung vorzugsweise ohne Reaktion mit Verbindungen, welche keine Isocyanatgruppen enthalten, stattfindet. Solche Modifizierungen sind insbesondere Dimerisierungen (Uretdionbildung), Trimerisierungen (Isocyanurat- sowie Iminooxadiazindionbildung) und/oder Oligomerisierungen unter Ausbildung von Spezies mit einheitlichen sowie verschiedenen Strukturtypen der vorstehend genannten Art im Oligomer- beziehungsweise Makromolekül.

Im Rahmen der vorliegenden Erfindung versteht man unter Polyisocyanaten Isocyanate mit zwei oder mehr Isocyanatgruppen im Molekül, also einer NCO-Funktionalität ≥ 2, die mindestens 2 Einheiten des monomeren Diisocyanates enthalten. Vorteilhafterweise kommen aliphatische cycloaliphatische oder araliphatische Diisocyanate in der Herstellung von Polyisocyanaten zum Einsatz. Insbesondere kann man aliphatische Diisocyanate verwenden, welche auch verzweigt sein können und/oder cyclische Reste enthalten können.

Die Wahl des Katalysators unterliegt zunächst keinen Einschränkungen, solange er für die gewünschte Modifizierungsreaktion geeignet ist.

Der Vorteil des erfindungsgemäßen Verfahrens liegt zum Einen darin, dass auch strukturell einfach aufgebaute und damit kostengünstige Katalysatoren eingesetzt werden können, selbst wenn sie weniger aktiv sein sollten und daher lange Reaktionszeiten benötigen. Im erfindungsgemäßen Der Vorteil des erfindungsgemäßen Verfahrens liegt zum Einen darin, dass auch strukturell einfach aufgebaute und damit kostengünstige Katalysatoren eingesetzt werden können, selbst wenn sie weniger aktiv sein sollten und daher lange Reaktionszeiten benötigen. Im erfindungsgemäßen Verfahren wurde zudem beobachtet, dass es zu einer weniger stark ausgeprägten Deaktivierung des Katalysators kommt. Hiedurch lassen sich längere Reaktionszeiten mit demselben Katalysator und eine gleichmäßigere Reaktionsführung darstellen.

Im Reaktionsansatz kann der Katalysator unverdünnt oder in einem Lösungsmittel gelöst eingebracht werden. Bei sehr aktiven Katalysatoren ist mitunter die Verdünnung vorteilhaft, um spontane Übervernetzungsreaktionen an der Dosierstelle des Katalysators zurück zu drängen, weniger aktive Katalysatoren werden vorteilhafterweise unverdünnt eingesetzt. Der Katalysator kann beispielweise in einem Anteil ≥ 0,0005 mol-% bis ≤ 5 mol-%, bevorzugt ≥ 0,001 mol-% bis ≤ 3 mol-%, bezogen auf die eingesetzte Menge an Diisocyanat, eingesetzt werden.

Erfindungsgemäß werden Diisocyanate eingesetzt, die aus einer Gasphasenphosgenierung erhalten wurden, wobei Quench-Flüssigkeit (Abkühl-Flüssigkeit) eingedüst wird. Solch ein Verfahren wird in DE 102 45 701 A1 beschrieben.

Bei dieser Herstellungsweise der Diisocyanate erreicht man durch das Versprühen einer geeigneten Quench-Flüssigkeit eine gewünschte rasche Abkühlung des Gasgemisches, welches ein Diisocyanat, Chlorwasserstoff und überschüssiges Phosgen umfasst. Beim Verlassen des Reaktors weist das Gasgemisch beispielsweise eine Temperatur zwischen ≥ 300°C und ≤ 400 °C auf und wird durch das Quenchen auf beispielsweise ≤ 150 °C abgekühlt. Die Kontaktzeit, in der die Abkühlung erfolgt, kann dabei ≥ 0,2 Sekunden bis ≤ 3 Sekunden sein.

Das Versprühen der Flüssigkeit kann mittels üblicher Sprühdüsen oder durch Öffnungen, beispielsweise Schlitze oder Löcher, am Ausgang der Reaktionszone und/oder am Eingang der Quench-Zone erfolgen. Sind nur zwei Sprühdüsen vorgesehen, so sind diese bevorzugt diametral angeordnet. Bei den Sprühdüsen kann es sich um Einzeldüsen handeln. Vorteilhafterweise werden jedoch Düsenköpfe mit jeweils mindestens zwei Einzeldüsen eingesetzt, wobei bevorzugt Einstoffdüsen gewählt werden.

Ein weiterer Askpekt dieser Herstellweise der Diisocyanate ist, dass die Quench-Flüssigkeit so in den Gasstrom eingesprüht wird, dass das heisse Reaktionsgas die relativ kalten Flächen der Quench-Zone oder der Düsen und deren Zuleitunge nicht kontaktiert. Erst wenn die Gasmischung auf den für das jeweilige Diisocyanat stabilen Temperaturbereich abgekühlt ist, kommt sie in Berührung mit den relativ kalten Wänden der Quench-Zone oder anderer Bauteile.

Die Sprühdüsen sind bevorzugt unabhängig voneinander so angeordnet, dass jeweils die Strömungsrichtung der Quench-Flüssigkeit einen Winkel von ≥ 0° bis ≤ 50°, insbesondere von ≥ 20° bis ≤ 35°, gegenüber der Strömungsrichtung des Gasgemisches aufweist. Die Strömungsrichtung des Gasgemisches verläuft im Wesentlichen entlang der Achse der zylinderförmigen Reaktionszone oder der Quench-Zone. Bei einer senkrechten Anordnung eines Rohrreaktors strömt das Gas von oben nach unten durch die Reaktionszone und die nachgeschaltete Quench-Zone. Analog verläuft die Strömungsrichtung der Quench-Flüssigkeit entlang der Achse der Sprühdüse. Der Öffnungswinkel der Sprühdüsen beträgt unabhängig voneinander bevorzugt von ≥ 20° bis ≤ 90°, besonders bevorzugt von ≥ 30° bis ≤ 60°. In einer besonders bevorzugten Variante weist die Strömungsrichtung all derjenigen Düsen, die in einer Ebene angeordnet sind, denselben Winkel gegenüber der Strömungsrichtung des Gasgemisches sowie denselben Öffnungswinkel auf.

In einer Ausführungsform des Verfahrens umfasst die Modifizierung der Diisocyanate die Bildung von Polyisocyanaten mit Uretdion-, Isocyanurat-, Iminooxadiazindion-, Polyamid-, Carbodiimid-, Uretonimin-, Urethan-, Allophanat-, Harnstoff-, Biuret-, Amid-, Acrylharnstoff-, Imid-, Oxazolidon-, Oximcarbamat- und/oder Oxadiazintrionstrukturen. Bevorzugt ist hierbei die sogenannte Dimerisierung oder Trimerisierung der Diisocyanate, das heisst die Modifizierung unter Ausbildung von Uretdion-, Isocyanurat- und/oder Iminooxadiazindionstrukturen.

In einer weiteren Ausführungsform des Verfahrens ist das Diisocyanat ausgewählt aus der Gruppe umfassend Hexamethylendiisocyanat, Isophorondiisocyanat, Toluylendiisocyanat und/oder Dicycylohexylmethandiisocyanat. Diese Diisocyanate haben in ihrer monomeren form vergleichsweise hohe Dampfdrucke und werden daher im Allgemeinen modifiziert.

In einer weiteren Ausführungsform des Verfahrens umfasst der Katalysator ein tertiäres Phosphan. Insbesondere kann der Katalysator Phosphane der Formel R¹R²R³P umfassen, wobei R¹, R² und R³ unabhängig voneinander für gleiche oder verschiedene linearaliphatische, verzweigt aliphatische sowie cycloaliphatische C₁ bis C₃₀ Reste stehen können. So kann R¹ ein cycloaliphatischer C₃ bis C₃₀-Rest sein und R² sowie R³ sind unabhängig voneinander ein cycloaliphatischer C₃ bis C₃₀-Rest oder ein linear oder verzweigt aliphatischer C₁ bis C₃₀-Rest. Die cycloaliphatischen C₃ bis C₃₀-Reste von R¹, R² und R³ können zusätzlich und unabhängig voneinander auch einfach oder mehrfach C₁ bis C₁₂ alkyl- oder alkoxysubstituiert sein. Bevorzugt ist R¹ ein Cyclopropyl-, Cyclobutyl-, Cyclopentyl- oder Cyclohexyl-Rest. Dieser Rest kann auch einfach oder mehrfach C₁ bis C₁₂ alkyl- oder alkoxysubstituiert sein.

Weiterhin bevorzugt sind R² und R³ unabhängig voneinander ein Cyclopropyl-, Cyclobutyl-, Cyclopentyl- oder Cyclohexyl-Rest oder ein aliphatischer C₂ bis C₈-Alkylrest. Diese cyclischen Reste können auch einfach oder mehrfach C₁ bis C₁₂ alkyl- oder alkoxysubstituiert sein.

Beispiele für einsetzbare Cycloalkylphosphane sind: Cyclopentyl-dimethylphosphan, Cyclopentyl-diethylphosphan, Cyclopentyl-di-n-propylphosphan, Cyclopentyl-di-isopropylphosphan, Cyclopentyl-dibutylphosphan, wobei "Butyl" für alle Isomeren, also n-Butyl, iso-Butyl, 2-Butyl, tert.-Butyl sowie cyclo-Butyl stehen kann, Cyclopentyl-dihexylphosphan (alle isomeren Hexylreste), Cyclopentyl-dioctylphosphan (alle isomeren Octylreste), Dicyclopentyl-methylphosphan, Dicyclopentylethylphosphan, Dicyclopentyl-n-propylphosphan, Dicyclopentyl-isopropylphosphan, Dicyclopentyl-butylphosphan (alle isomeren Butylreste), Dicyclopentyl-hexylphosphan (alle isomeren Hexylreste), Dicyclopentyl-octylphosphan (alle isomeren Octylreste), Tricyclopentylphosphan, Cyclohexyl-dimethylphosphan, Cyclohexyl-diethylphosphan, Cyclohexyl-di-n-propylphosphan, Cyclohexyl-di-isopropylphosphan, Cyclohexyl-dibutylphosphan (alle isomeren Butylreste), Cyclohexyl-dihexylphosphan (alle isomeren Hexylreste), Cyclohexyl-dioctylphosphan (alle isomeren Octylreste), Dicyclohexyl-methylphosphan, Dicyclohexyl-etylphosphan, Dicyclohexyl-n-propylphosphan, Dicyclohexyl-isopropylphosphan, Dicyclohexyl-butylphosphan (alle isomeren Butylreste), Dicyclohexyl-hexylphosphan (alle isomeren Hexylreste), Dicyclohexyl-octylphosphan (alle isomeren Octylreste), und Tricyclohexylphosphan.

Diese können als Modifizierungskatalysator einzeln, in beliebigen Mischungen untereinander oder in Mischungen mit anderen primären, sekundären und/oder tertiären Alkyl-, Aralkyl- und/oder Arylphosphanen verwendet werden.

In einer weiteren Ausführungsform umfasst der Katalysator Dicyclopentyl-n-butylphosphan. Diese Verbindung ist gut zugänglich und hat im Experiment gezeigt, dass im erfindungsgemäßen Verfahren die Aktivität lange erhalten bleibt. Weiterhin kann sie aufgrund ihres Siedepunkts zusammen mit nicht reagiertem Diisocyanat von der Reaktionsmischung entfernt werden, so dass kein Katalysator im erhaltenen Harz zurückbleibt.

In noch einer anderen Ausführungsform umfasst der Katalysator Phosphane der Formel R⁴R⁵R⁶P, wobei R⁴ ein bicyclischer aliphatischer C₆ bis C₃₀-Rest ist und R⁵ sowie R⁶ unabhängig voneinander ein mono- oder bicyclischer aliphatischer C₄ bis C₃₀-Rest oder ein linear oder verzweigt aliphatischer C₁ bis C₃₀-Rest sind. Die cycloaliphatischen C₄ bis C₃₀-Reste von R⁴, R⁵ und R⁶ können unabhängig voneinander auch einfach oder mehrfach C₁ bis C₁₂ alkyl- oder alkoxysubstituiert sein.

Bevorzugt sind hierbei Verbindungen, bei denen R⁴ für einen ein- oder mehrfach C₁ bis C₁₂ alkylsubstituierten Norbomylrest (2,2,1-Bicycloheptylrest) steht und R⁵ wahlweise identisch zu R⁴ oder zu R⁶ ist und wobei R⁶ für einen ein- oder mehrfach C₁ bis C₈ alkylsubstituierten, aliphatischen C₁ bis C₁₂-Alkylrest steht.

Beispiele für einsetzbare Phosphate sind: Norbornyl-dimethylphosphan, Norbornyl-diethylphosphan, Norbornyl-di-n-propylphosphan, Norbornyl-di-isopropylphosphan, Norbornyl-dibutylphosphan wobei 'Butyl' für alle Isomeren, also n-Butyl, iso-Butyl, 2-Butyl, tert.-Butyl sowie cyclo-Butyl stehen kann, Norbornyl-dihexylphosphan (alle isomeren Hexylreste), Norbornyl-dioctylphosphan (alle isomeren Octylreste), Dinorbornyl-methylphosphan, Dinorbornyl-ethylphosphan, Dinorbornyl-n-propylphosphan, Dinorbornyl-isopropylphosphan, Dinorbornyl-butylphosphan (alle isomeren Butylreste), Dinorbornyl-hexylphosphan (alle isomeren Hexylreste), Dinorbornyl-octylphosphan (alle isomeren Octylreste), Trinorbomylphosphan, Dimethyl-(1,7,7-trimethyl-bicyclo[2.2.1]hept-2-yl)-phosphan, Diethyl-(1,7,7-trimethyl-bicyclo[2.2.1]hept-2-yl)-phosphan, Di-n-propyl-(1,7,7-timethyl-bicyclo[2.2.1]hept-2-yl)-phosphan, Di-iso-propyl-(1,7,7-trimethyl-bicyclo[2.2.1]hept-2-yl)-phosphan, Dibutyl-(1,7,7-timethyl-bicyclo[2.2.1]hept-2-yl)-phosphan (alle isomeren Butylreste), Dihexyl-(1,7,7-timethyl-bicyclo[2.2.1]hept-2-yl)-phosphan (alle isomeren Hlexylreste), Dioctyl-(1,7,7-timethyl-bicyclo[2.2.1]hept-2-yl)-phosphan (alle isomeren Octylreste), Methyl-bis-(1,7,7-trimethyl-bicyclo[2.2.1]hept-2-yl)-phosphan, Ethyl-bis-(1,7,7-timethyl-bicyclo[2.2.1]hept-2-yl)-phosphan, n-Propyl-bis-(1,7,7-timethyl-bicyclo[2.2.1]-hept-2-yl)-phosphan, iso-Propyl-bis-(1,7,7-trimethyl-bicyclo[2.2.1]hept-2-yl)-phosphan, Butyl-bis-(1,7,7-timethyl-bicyclo[2.2.1]hept-2-yl)-phosphan (alle isomeren Butylreste), Hexyl-bis-(1,7,7-trimethyl-bicyclo[2.2.1]hept-2-yl)-phosphan (alle isomeren Hexylreste), Octyl-bis-(1,7,7-trimethyl-bicyclo[2.2.1]hept-2-yl)-phosphan (alle isomeren Octylreste), Dimethyl-(2,6,6-timethyl-bicyclo-[3.1.1]hept-3-yl)-phosphan, Diethyl-(2,6,6-trimethyl-bicyclo[3.1:1]hept-3-yl)-phosphan, Di-n-propyl-(2,6,6-trimethyl-bicyclo[3.1.1]hept-3-yl)-phosphan, Di-iso-propyl-(2,6,6-timethyl-bicyclo-[3.1.1]hept-3-yl)-phosphan, Dibutyl-(2,6,6-timethyl-bicyclo[3.1.1]hept-3-yl)-phosphan (alle isomeren Butylreste), Dihexyl-(2,6,6-timethyl-bicyclo[3.1.1]hept-3-yl)-phosphan (alle isomeren Hexylreste), Dioctyl-(2,6,6-timethyl-bicyclo[3.1.1]hept-3-yl)-phosphan (alle isomeren Octylreste), Methyl-bis-(2,6,6-trimethyl-bicyclo[3.1.1]hept-3-yl)-phosphan, Ethyl-bis-(2,6,6-trimethy)-bicyclo[3.1.1]hept-3-yl)-phosphan, n-Propyl-bis-(2,6,6-timethyl-bicyclo[3.1.1]hept-3-yl)-phosphan, iso-Propyl-bis-(2,6,6-timcthyl-bicyclo[3.1.1]hept-3-yl)-phosphan, Butyl-bis-(2,6,6-trimethyl-bicyclo[3.1.1]hept-3-yl)-phosphan (alle isomeren Butylreste), Hexyl-bis-(2,6,6-trimethyl-bicyclo[3.1.1]hept-3-yl)-phosphan (alle isomeren Hexylreste), sowie Octyl-bis-(2,6,6-trimethyl-bicyclo[3.1.1]hept-3-yl)-phosphan (alle isomeren Octylreste).

Die vorgenannten Verbindungen können als Modifizierungskatalysator einzeln, in beliebigen Mischungen untereinander oder in Mischungen mit anderen primären, sekundären und/oder tertiären Alkyl-, Aralkyl- und/oder Arylphosphanen verwendet werden.

In einer weiteren Ausführungsform des Verfahrens wird die Modifizierung der Diisocyanate im kontinuierlichen Betrieb durchgeführt. Dieses bedeutet, dass mindestens eine Komponente der Reaktionsmischung in einem Kreislauf gefahren wird, so dass mehr als ein Reaktionslauf durchgeführt werden kann. Vorteilhaftweise wird der kontinuierliche Betrieb so durchgeführt, dass das nicht modifizerte Diisocyanat zusammen mit dem Katalysator von dem modifizierten Polyisocyanat abgetrennt werden. Die Mischung aus nicht modifiziertem Diisocyanat und aufgrund der im erfindungsgemäßen Verfahren eingesetzten Diisocyanate nicht oder nur im Vergleich zu bekannten Verfahren weniger stark deaktiviertem Katalysator kann wieder mit frischem nicht modifiziertem Diisocyanat aufgefüllt werden und erneut zur Reaktion gebracht werden. In einer bevorzugten Variante werden nicht modifiziertes Diisocyanat und Katalysator abdestilliert, wobei das modifizierte Polyisocyanat als Sumpfprodukt zurückbleibt.

In einer weiteren Ausführungsform des Verfahrens ist die Quench-Flüssigkeit ausgewählt aus der Gruppe umfassend Toluol, Monochlortoluol, Xylol, Monochlornaphthalin, Monochlorbenzol und/oder Dichlorbenzol. Insbesondere eignen sich Monochlorbenzol und ortho-Dichlorbenzol. Auch kann eine Lösung des gebildeten Diisocyanats in einem dieser organischen Lösungsmittel verwendet werden. Dabei beträgt der Lösungsmittelanteil vorzugsweise ≥ 40 Volumen-% bis ≤ 90 Volumen-%. Allgemein beträgt die Temperatur der Quench-Flüssigkeit vorzugsweise ≥ 100 °C bis ≤ 170 °C.

In einer weiteren Ausführungsform des Verfahrens sind die Reaktionszone und/oder Quench-Zone zylinderförmig ausgeführt. Dieses ist apparativ leicht zu realisieren und es treten keine thermischen Inhomogenitäten auf, wenn das Reaktionsgemisch vor oder nach dem Quenchen in Kontakt mit Behälterwänden kommt. Bei der Reaktionszone handelt es sich vorzugsweise um einen Rohrreaktor ohne Einbauten. Weiterhin ist es möglich, dass der Durchmesser der Quench-Zone größer ist als der Durchmesser der Reaktionszone ist.

Ein weiterer Gegenstand der vorliegenden Erfindung sind modifizierte Polyisocyanate, erhalten durch das erfindungsgemäße Verfahren. Beispiele für Modifikationsreaktionen und für ausgewählte Polyisocyanate wurden bereits vorstehend ausgeführt.

Noch ein weiterer Gegenstand der vorliegenden Erfindung ist die Verwendung von erfindungsgemäßen modifizierten Polyisocyanaten zur Herstellung von Formkörpern, geschäumten Formkörpern, Lacken, Beschichtungsmitteln, Klebstoffen, Dichtmassen und/oder Zuschlagstoffen. In den Polyisocyanaten können die enthaltenen freien, nicht modifizierten NCO-Gruppen gegebenenfalls auch blockiert sein. Als Blockierungsmittel können insbesondere Phenole (zum Beispiel Phenol, Nonylphenol, Kresol), Oxime (zum Beispiel Butanonoxim, Cyclohexanonoxim), Lactame (zum Beispiel ε-Caprolactam), sekundäre Amine (zum Beispiel Diisopropylamin), Pyrazole (zum Beispiel Dimethylpyrazol), Imidazole, Triazole oder Malon- und Essigsäureester verwendet werden.

Die nach dem erfindungsgemäßen Verfahren hergestellten Polyisocyanate können insbesondere zur Herstellung von Ein- und Zweikomponenten-Polyurethanlacken eingesetzt werden. Sie können gegebenenfalls in Mischungen mit anderen Di- oder Polyisocyanaten wie Biuret-, Urethan-, Allophanat-, Isocyanurat-, sowie Iminooxadiazindiongruppen enthaltenden Di- oder Polyisocyanaten vorliegen. Ebenfalls möglich ist die Verwendung der erfindungsgemäßen Polyisocyanate auf Basis gegebenenfalls verzweigter, linearaliphatischer Diisocyanate als Reaktivverdünner zur Viskositätserniedrigung höherviskoser oder bei Zimmertemperatur fester Polyisocyanat-Harze.

Die erwähnten Beschichtungsmittel können in Lösung oder aus der Schmelze sowie bei Pulverlacken in fester Form mit Methoden wie Streichen, Rollen, Gießen, Spritzen, Tauchen, Wirbelschichtverfahren oder durch elektrostatische Sprühverfahren auf dem zu beschichtenden Gegenstand appliziert werden. Als Substrate eigenen sich beispielsweise Werkstoffe wie Metalle, Holz, Kunststoffe oder Keramiken.

Die Herstellung der erfindungsgemäß eingesetzten Diisocyanate wird anhand der nachfolgenden Zeichnung weiter beschrieben. Es zeigt:

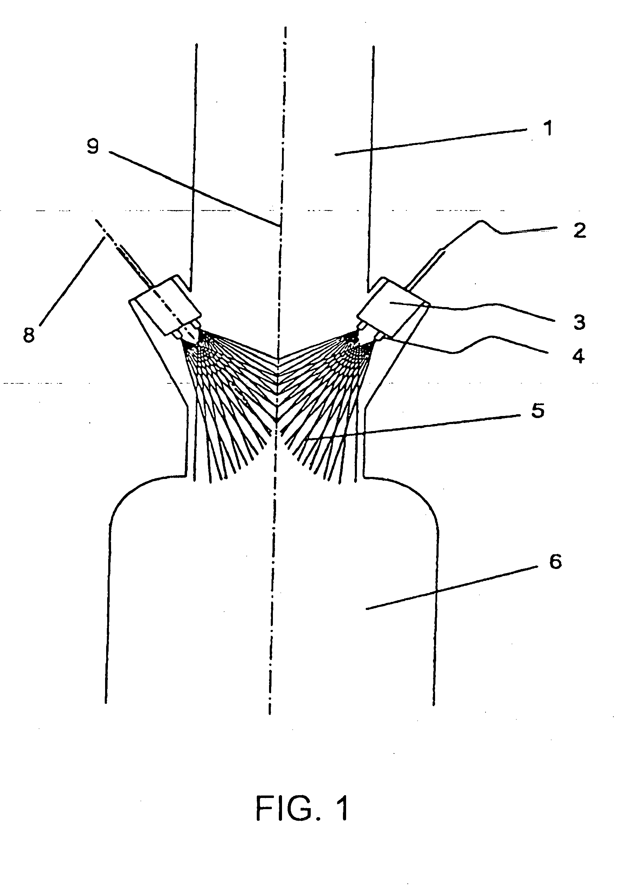

FIG. 1 einen schematischen Querschnitt einer Reaktionszone und Quench-Zone

FIG. 1 zeigl eine zylinderförmige Reaktionszone 1, die von dem gasförmigen Gemisch von oben nach unten entlang der gestrichelten Linie 9 durchströmt wird. Beim Verlassen der Reaktionszone 1 durchströmt das Gasgemisch eine ebenfalls zylinderförmige Quench-Zone 5. In der Quench-Zone 5 sind zwei Düsenköpfe 3 mit je zwei Einzeldüsen 4 diametral angeordnet. Die Quench-Flüssigkeit wird über eine Zuleitung 2 dem Düsenkopf 3 zugefiührt. Die Düsen 4 beziehungsweise der Düsenkopf 3 sind so angeordnet, dass die Strömungsrichtung der Quench-Flüssigkeit (dargestellt durch die gestrichelte Linie 8) und die des Gasstroms 9 einen Winkel von 0° bis 50°, insbesondere von 20° bis 35°, zueinander aufweisen und damit das heisse Gasgemisch die kälteren Düsen oder den Düsenkopf nicht kontaktiert. In der Quench-Zone 5 erfolgt die Abkühlung des Reaktionsgases durch Verdampfung der zerstäubten Flüssigkeit. Die verbleibende Flüssigkeit und das abgekühlte Reaktionsgas gelangen in den darunter liegenden Flüssigkeitssammelbehälter 6, der gleichzeitig als Pumpenvorlage und als Trennapparat für Gas und Flüssigkeit dient.

Die vorliegende Erfindung wird weiterhin anhand des nachfolgenden erfindungsgemäßen Beispiels und des Vergleichsbeispiels erläutert. Hierbei ist zur Veranschaulichung das System HDI als zu modifizierendes Diisocyanat/teriäres. Phosphan als Katalysator gewählt worden, was nicht bedeuten soll, dass die vorliegende Erfindung auf dieses Diisocyanat oder diesen Katalysatortyp beschränkt sein soll.

In den Beispielen sind alle Prozentangaben, soweit nicht anders vermerkt, als Gewichtsprozent zu verstehen. Die Reaktionen wurden mit frisch entgastem Hexamethylendiisocyanat (HDI) als Edukt durchgeführt. Die Bezeichnung "entgast" bedeutet dabei, dass das eingesetzte HDI unmittelbar vor der katalytischen Umsetzung durch mindestens 30-minütiges Rühren im Vakuum (<1 mbar) von gelösten Gasen, insbesondere CO₂, befreit und anschließend mit Stickstoff belüftet wird.

Alle Reaktionen wurden unter einer trockenen Stickstoffatmosphäre durchgeführt. Die in den Beispielen beschriebenen Chemikalien und Katalysatoren wurden von der Firma Bayer (HDI) oder Cytec (Phosphan) erhalten und sind ohne weitere Reinigung eingesetzt worden.

### Beispiel 1 (erfindungsgemäßes Beispiel)

In einem doppelwandigen, durch einen externen Kreislauf auf 30 °C temperierten Planschliffgefäß mit Rührer sowie einem an eine Inertgasanlage (Stickstoff/Vakuum) angeschlossenen Rückflusskühler und Thermometer wurden 1050 g HDI, hergestellt gemäß DE 102 45 704 A1, vorgelegt und entgast. Nach Belüften mit Stickstoff wurden 12 g Dicyclopentyl-n-butylphosphan zugesetzt und es wurde Für etwa 24 Stunden bei 30 °C gerührt. Anschließend wurde das Reaktionsgemisch ohne vorherige Desaktivierung des Katalysators aufgearbeitet. Die Aufarbeitung erfolgte durch Vakuumdestillation (0,08 mbar) in einem Dünnschichtverdampfer vom Typ Kurzwegverdampfer (KWV; Temperatur des Heizmediums: 150°C) mit vorgeschaltetem Vorverdampfer (VV, Temperatur des Heizmediums: 120 °C), wobei nicht umgesetztes Monomer gemeinsam mit dem aktiven Katalysator als Destillat und das Polyisocyanatharz als Sumpfprodukt separiert wurden.

Dieser Reaktionsansatz war der Startansatz mit der Versuchsnummer 1-0 gemäß der nachfolgenden Tabelle 1.

Das den aktiven Katalysator enthaltende Destillat wurde in eine zweite Planschliff-Rührapparatur, die identisch zur eingangs genannten aufgebaut war, dosiert und unmittelbar nach Destillationsende mit frisch entgastem HDI auf die Ausgangsmenge (1050 g) aufgefüllt. Anschließend wurde erneut für etwa 24 Stunden bei 30 °C gerührt und aufgearbeitet wie oben beschrieben. Dieser Reaktionsansatz trägt in der Tabelle 1 die Versuchsnummer 1-A.

Diese Verfahrensweise wurde insgesamt 24 mal wiederholt (bis Versuchsnummer 1-X in Tabelle 1), wobei die Versuche 1-E, 1-J, 1-O und 1-T zur Realisierung höherer Harzausbeuten etwa 72 Stunden liefen und dann erst aufgearbeitet wurden.

Man beobachtete im Verlauf der über mehrere Wochen durchgeführten Versuchsserie eine nur langsame Abnahme der katalytischen Aktivität, wobei als Maß für deren Abnahme die sinkende Harzausbeute herangezogen wurde. Die relative Aktivität des Katalysators wurde berechnet, indem die im Reaktionsansatz erhaltene Harzausbeute durch 1050 g, also dem Gewicht des eingesetzten und zu modifizierenden HDI, dividiert wurde und dann zu der Harzausbeute im Startansatz (definiert als 100% relative Aktivität) ins Verhältnis gesetzt wurde.

**Tabelle 1 (erfindungsgemäßes Beispiel)**

| Versuchsnummer | Relative Aktivität [%] | Versuchsnummer | Relative Aktivität [%] |
|---|---|---|---|
| 1-0 | 100 | 1-M | 82 |
| 1-A | 100 | 1-N | 75 |
| 1-B | 97 | 1-O | 56 |
| 1-C | 97 | 1-P | 80 |
| 1-D | 99 | 1-Q | 68 |
| 1-E | 80 | 1-R | 66 |
| 1-F | 100 | I-S | 66 |
| 1-G | 79 | 1-T | 55 |
| 1-H | 76 | 1-U | 71 |
| 1-I | 74 | 1-V | 74 |
| 1-J | 64 | 1-W | 76 |
| 1-K | 92 | 1-X | 71 |
| 1-L | 81 | | |

### Beispiel 2 (Vergleichsbeispiel)

Es wurde wie in Beispiel 1 beschrieben verfahren, jedoch mit dem Unterschied, dass nicht gemäß DE 102 45 704 A1 hergestelltes HDI zum Einsatz kam. Das HD1 wurde nach einem Phosgenierverfahren gemäß EP 02 89 840 erhalten und wies den gleichen Gehalt an hydrolysierbarem Chlor (HC-Gehalt) auf wie das HDI aus Beispiel 1, nämlich 22 ppm. Die Ergebnisse sind in Tabelle 2 zusammengefasst. Wie man erkennt, nimmt hier die Aktivität des Katalysators bei fortlaufender Recyclierung des Monomers deutlich schneller ab als im erfindungsgemäßen Bespiel.

**Tabelle 2 (Vergleichsbeispiel)**

| Versuchsnummer | Relative Aktivität [%] | Versuchsnummer | Relative Aktivität [%] |
|---|---|---|---|
| 2-0 | 100 | 2-M | 65 |
| 2-A | 98 | 2-N | 61 |
| 2-B | 97 | 2-O | 49 |
| 2-C | 96 | 2-P | 55 |
| 2-D | 94 | 2-Q | 51 |
| 2-E | 78 | 2-R | 45 |
| 2-F | 88 | 2-S | 39 |
| 2-G | 75 | 2-T | 28 |
| 2-H | 72 | 2-U | 33 |
| 2-I | 70 | 2-V | 31 |
| 2-J | 59 | 2-W | 26 |
| 2-K | 75 | 2-X | 24 |
| 2-L | 69 | | |

## Patentansprüche

1. Verfahren zur Modifizierung von Diisocyanaten zur Herstellung von Polyisocyanaten, wobei das Diisocyanat unter Verwendung eines Katalysators umgesetzt wird und wobei die Modifizierung der Diisocyanate die Bildung von Polyisocyanaten mit Uretdion-, Isocyanurat-, Iminooxadiazindion-, Polyamid-, Carbodiimid-, Uretonimin-, Urethan-, Allophanat-, Harnstoff-, Biuret-, Amid-, Acrylharnstoff , Imid-, Oxazolidon-, Oximcarbamat- und/oder Oxadiazintrionstrukturen betrifft, **dadurch gekennzeichnet, dass** das eingesetzte Diisocyanat erhalten wird, indem
bei der Phosgenierung von Diaminen in der Gasphase zur Herstellung von Diisocyanaten das gasförmige Reaktionsgemisch gequencht wird,
wobei das gasförmige Reaktionsgemisch wenigstens ein Diisocyanat, Phosgen und Chlorwasserstoff umfasst,
wobei eine Quench-Flüssigkeit in das kontinuierlich aus einer Reaktionszone (1) in eine nachgeschaltete Quench-Zone (5) strömende Gasgemisch eingedüst wird und
wobei die Quench-Flüssigkeit mit Hilfe von mindestens zwei Sprühdüsen (4), welche am Eingang der Quench-Zone (5) in gleichen Abständen entlang des Umfangs der Quench-Zone (5) angeordnet sind, eingedüst wird.

2. Verfahren gemäß Anspruch 1, wobei das Diisocyanat ausgewählt ist aus der Gruppe umfassend Hexamethylendiisocyanat, Isophorondiisocyanat, Toluylendiisocyanat und/oder Dicyclohexylmethandiisocyanat.

3. Verfahren gemäß Anspruch 1, wobei der Katalysator ein tertiäres Phosphan umfasst.

4. Verfahren gemäß Anspruch 3, wobei der Katalysator Dicyclopentyl-n-butylphosphan umfasst.

5. Verfahren gemäß Anspruch 1, wobei die Modifizierung der Diisocyanate im kontinuierlichen Betrieb durchgeführt wird.

6. Verfahren gemäß Anspruch 1, wobei die Quench-Flüssigkeit ausgewählt ist aus der Gruppe umfassend Toluol, Monochlortoluol, Xylol, Monochlornaphthalin, Monochlorbenzol und/oder Dichlorbenzol.

7. Verfahren gemäß Anspruch 1, wobei die Reaktionszone (1) und/oder Quench-Zone (5) zylinderförmig ausgeführt sind.

## Claims

1. Process for modifying diisocyanates for preparing polyisocyanates, where the diisocyanate is reacted using a catalyst and the modification of the diisocyanates involves formation of polyisocyanates having uretdione, isocyanurate, iminooxadiazinedione, polyamide, carbodiimide, uretonimine, urethane, allophanate, urea, biuret, amide, acrylurea, imide, oxazolidone, oxime carbamate and/or oxadiazinetrione structures **characterized in that** the diisocyanate used is obtained by quenching the gaseous reaction mixture in the phosgenation of diamines in the gas phase,
where the gaseous reaction mixture comprises at least a diisocyanate, phosgene and hydrogen chloride,
a quenching liquid is sprayed into the gas mixture which flows continuously from a reaction zone (1) into a downstream quenching zone (5) and
the quenching liquid is sprayed in by means of at least two spray nozzles (4) which are arranged at the inlet of the quenching zone (5) at equal intervals along the circumference of the quenching zone (5).

2. Process according to Claim 1, wherein the diisocyanate is selected from the group consisting of hexamethylene diisocyanate, isophorone diisocyanate, tolylene diisocyanate and dicyclohexylmethane diisocyanate.

3. Process according to Claim 1, wherein the catalyst comprises a tertiary phosphane.

4. Process according to Claim 3, wherein the catalyst comprises dicyclopentyl-n-butylphosphane.

5. Process according to Claim 1, wherein the modification of the diisocyanates is carried out in continuous operation.

6. Process according to Claim 1, wherein the quenching liquid is selected from the group consisting of toluene, monochlorotoluene, xylene, monochloronaphthalene, monochlorobenzene and dichlorobenzene.

7. Process according to Claim 1, wherein the reaction zone (1) and/or the quenching zone (5) are cylindrical.

## Revendications

1. Procédé de modification de diisocyanates pour la fabrication de polyisocyanates, le diisocyanate étant mis en réaction en utilisant un catalyseur et la modification des diisocyanates concernant la formation de polyisocyanates contenant des structures uretdione, isocyanurate, iminooxadiazinedione, polyamide, carbodiimide, uretonimine, uréthane, allophanate, urée, biuret, amide, acrylurée, imide, oxazolidone, carbamate d'oxime et/ou oxadiazinetrione, **caractérisé en ce que** le diisocyanate utilisé est obtenu par
désactivation du mélange réactionnel gazeux lors de la phosgénation de diamines en phase gazeuse pour la fabrication de diisocyanates,
le mélange réactionnel gazeux comprenant au moins un diisocyanate, du phosgène et du chlorure d'hydrogène, un liquide de désactivation étant injecté dans le mélange gazeux qui s'écoule en continu depuis une zone de réaction (1) vers une zone de désactivation (5) en aval, et
le liquide de désactivation étant injecté à l'aide d'au moins deux buses de pulvérisation (4), qui sont agencées à l'entrée de la zone de désactivation (5) à des distances égales le long de la périphérie de la zone de désactivation (5).

2. Procédé selon la revendication 1, dans lequel le diisocyanate est choisi dans le groupe comprenant le diisocyanate d'hexaméthylène, le diisocyanate d'isophorone, le diisocyanate de toluylène et/ou le diisocyanate de dicyclohexylméthane.

3. Procédé selon la revendication 1, dans lequel le catalyseur comprend un phosphane tertiaire.

4. Procédé selon la revendication 3, dans lequel le catalyseur comprend du dicyclopentyl-n-butylphosphane.

5. Procédé selon la revendication 1, dans lequel la modification des diisocyanates est réalisée en mode continu.

6. Procédé selon la revendication 1, dans lequel le liquide de désactivation est choisi dans le groupe comprenant le toluène, le monochlorotoluène, le xylène, la monochloronaphtaline, le monochlorobenzène et/ou le dichlorobenzène.

7. Procédé selon la revendication 1, dans lequel la zone de réaction (1) et/ou la zone de désactivation (5) sont configurées sous forme cylindrique.
